# EUROPÄISCHE PATENTSCHRIFT

(11) **EP 1 070 513 B1**
(45) Veröffentlichungstag und Bekanntmachung des Hinweises auf die Patenterteilung: **14.06.2006**
(21) Anmeldenummer: 00250236.7
(22) Anmeldetag: 13.07.2000
(51) Int. Cl.: A61M 29/02

(54) **Ballonkatheter für Gefässabzweigungen**
Balloon catheter for branched vessels
Cathéter à ballonnet pour des vaisseaux ramifiés

(30) Priorität: 20.07.1999 DE 19934923
(43) Veröffentlichungstag der Anmeldung: 24.01.2001
(73) Patentinhaber: BIOTRONIK GmbH & Co. KG, 12359 Berlin (DE)
(72) Erfinder: Zedler, Stephan, 12309 Berlin (DE); Trip, Erik, 14823 Grubo (DE); Loos, Hartmut, 3090 Overijse (BE)
(74) Vertreter: Lindner-Vogt, Karin L.

(56) Entgegenhaltungen:
- EP-A- 0 897 700
- WO-A-97/13471
- DE-A- 3 915 289
- US-A- 5 672 153

## Beschreibung

Die Erfindung betrifft System zum Aufweiten verengter Gefäße im Bereich von Gefäßverzweigungen, insbesondere der Koronargefäße, das einen Ballonkatheter und einen auf dessen Ballon angeordneten Stent umfasst.

Ballonkatheter werden zum Aufweiten verengter Gefäße des menschlichen und tierischen Körpers verwendet. Der am distalen Ende des Katheterschafts angeordnete Ballon wird hierzu in der Regel entfernt von der Verengung in das Gefäßsystem eingeführt und in diesem an die Engstelle herangeführt. Anschließend wird der Ballon zum Aufweiten der Engstelle durch Druckbeaufschlagung expandiert.

Häufig ist auf dem Ballon ein rohrförmiges intraluminales Stützelement, ein sog. Stent, angeordnet, das bei der Expansion des Ballons unter plastischer Verformung radial ausgedehnt wird. Während der Ballon wieder entfernt wird, verbleibt der Stent in seinem ausgedehnten Zustand im Gefäß und hält so das Gefäß dauerhaft aufgeweitet.

Im Bereich von Gefäßverzweigungen, insbesondere der Koronargefäße, ist es besonders dann, wenn ein Stent implantiert werden soll, erforderlich den Ballon möglichst genau bezüglich der Gefäßverzweigung zu plazieren. Hierzu ist in der Regel ein im Bereich des Ballons angeordnetes erstes Führungsmittel vorgesehen, das zur Positionierung des Ballons im Bereich einer Gefäßverzweigung in den seitlich abzweigenden Gefäßast quer zur Längsrichtung des Ballonkatheters eingeführt werden kann und so als Positionierhilfe dient.

Ein solcher Ballonkatheter ist beispielsweise aus der europäischen Patentanmeldung EP 0 904 745 A2 bekannt, bei der ein seitlich in den abzweigenden Gefäßast einführbarer Führungsdraht entlang der Außenseite des Ballons verschieblich angeordnet ist.

Der bekannte Ballonkatheter weist jedoch den Nachteil auf, daß durch den auf der Außenseite des Ballons angeordneten, von einem Führungsschlauch umgebenen Führungsdraht bei der Expansion des Ballons eine in Umfangsrichtung ungleichmäßige Last in das Gefäß bzw. den gegebenenfalls vorhandenen Stent eingeleitet wird. Diese rührt von der Ausbauchung gegenüber der Ballonkontur her, die sich im Bereich des Führungsdrahtes bildet. Diese Ausbauchung führt zu einer unerwünschten in Umfangsrichtung ungleichmäßigen Deformation der anliegenden Gefäßwand bzw. des zwischen Ballon und Gefäßwand liegenden Stents. Hierbei ergeben sich im Bereich des Führungsdrahtes relativ kleine Krümmungsradien, d. h. ein relativ hoher Deformationsgrad. Dies kann sich auf eine geschwächte Gefäßwand, insbesondere aber auf die filigrane Struktur eines solchen Stents nachteilig auswirken. Es kann sogar zu lokalen Brüchen der Stentstruktur kommen, die unbedingt zu vermeiden sind. Ein weiterer Nachteil des bekannten Ballonkatheters liegt in der relativ großen Querabmessung des Ballons mit dem auf seiner Außenseite aufgebrachten Führungsdraht samt Führungsschlauch.

WO 97/13471 beschreibt unter anderem einen Ballonkatheter mit zwei Kammern zwischen denen eine Austrittsöffnung eines Führungsdrahtes liegt.

Der Erfindung liegt die Aufgabe zugrunde, ein System zum Aufweiten verengter Gefäße im Bereich von Gefäßverzweigungen, insbesondere der Koronargfeäße, das einen Ballonkatheter und einen auf dessen Ballon angeordneten Stent umfasst, bereitzustellen.

Die Aufgabe wird durch ein System zum Aufweiten verengter Gefäße, insbesondere verengter Koronargefäße, im Bereich vonGefäßverzweigungen nach Anspruch 1 gelöst, Das Systems umfasst dazu einen Ballonkatheter zum Einsatz im Bereich von Gefäßverzweigungen, insbesondere der Koronargefäße, mit einem Katheterschaft, an dessen distalem Ende wenigstens ein Ballon und wenigstens ein in dessen Bereich angeordnetes erstes Führungsmittel vorgesehen sind, wobei das erste Führungsmittel zur Positionierung des Ballons im Bereich einer Gefäßverzweigung in den seitlich abzweigenden Gefäßast quer zur Längsrichtung des Ballonkatheters einführbar ausgebildet ist, und der Ballon wenigstens zwei in Längsrichtung des Ballonkatheters voneinander beabstandete Kammern aufweist, zwischen denen das erste Führungsmittel angeordnet ist. Das Systems umfasst ferner einen auf den Ballon angeordneten Stent, der in dem an daß erste Führungsmittel angrenzenden Bereich zwischen den Kammern wenigstens einen am Umfang des Stentmantels angeordneten Verzweigungsabschnitt aufweist, der wenigstens ein zum Abstützen des Gefäßübergangs im Bereich einer Gefäßverzweigung vorgesehenes, im wesentlichen radial aus der Mantelfläche herausklappbares erstes Stützelement umfaßt und das erste Führungsmittel zum Herausklappen des Stützelements aus der Mantelfläche ausgebildet ist. Hiermit ist ein System zum Aufweiten verengter Gefäße zur Verfügung gestelit, das im Bereich von Gefäßverzweigungen ohne weitere Hilfsmittel eine zuverlässige Aufweitung des Gefäßes einschließlich einer Abstützung des Überganges in den abzweigenden Gefäßast sicherstellt.

Der Begriff Ballon wird hier im weiteren Sinne gebraucht und soll auch eine auf mehrere Ballons im engeren Sinne verteilte Anordnung von aufblasbaren, insbesondere aufblasbaren Kammern umfassen. In diesem Sinn können die beiden Kammern auch auf zwei Ballons im engeren Sinne verteilt sein.

Die Erfindung schließt die technische Lehre ein, daß man eine in Umfangsrichtung des Ballons gleichförmige Verformung der angrenzenden Elemente bei der Expansion erhält, wenn man das Führungsmittel nicht auf der Außenseite des Ballons anordnet. Bei der Expansion übt der im wesentlichen kreiszylindrische Ballon dann in Umfangsrichtung sowohl eine im wesentlichen gleichmäßige Verformungskraft als auch eine im wesentlichen gleichmäßige Deformation aus.

Um die einfache Positionierung mit Hilfe des ersten Führungsmittels weiterhin zu gewährleisten, weist der Ballon erfindungsgemäß wenigstens zwei in Längsrichtung des Ballonkatheters voneinander beabstandete Kammern auf, zwischen denen das erste Führungsmittel angeordnet ist.

Hierdurch ist auch eine kleinere Querabmessung des Ballons möglich, da eventuell erforderliche Leit- oder Zufuhrmittel für das zwischen den Kammern angeordnete Führungsmittel, beispielsweise ein Führungsschlauch für einen Führungsdraht, in den Katheterschaft integriert werden können und somit weniger Bauraum benötigt wird.

Das erste Führungsmittel kann dabei von einer beliebigen Einheit gebildet sein, die geeignet ist, zur Unterstützung der Positionierung des Ballonkatheters in den abzweigenden Gefäßast eingeführt zu werden. Es könnte beispielsweise von einem weiteren Ballon gebildet sein, der sich durch Befüllen in den abzweigenden Gefäßast hinein erstreckt. Vorzugsweise ist das erste Führungsmittel von einem ersten Führungsdraht gebildet, da sich hiermit eine besonders einfach handhabbare Konfiguration ergibt.

Der erste Führungsdraht ist weiter vorzugsweise in dem Katheterschaft geführt, wobei der Katheterschaft zwischen den beiden Kammern eine seitliche Austrittseinrichtung für den ersten Führungsdraht aufweist. Hierdurch ergibt sich eine besonders einfache und platzsparende Gestaltung des erfindungsgemäßen Ballonkatheters.

Bei vorteilhaften, weil einfach und vielseitig einsetzbaren Varianten der Erfindung ist ein zweiter Führungsdraht zum Führen des Ballonkatheters in seiner Längsrichtung vorgesehen. Dieser ist vorzugsweise ebenfalls in dem Katheterschaft geführt, wobei der Katheterschaft dann an seinem distalen Ende eine distale Austrittseinrichtung für den zweiten Führungsdraht aufweist.

Die Führungsmittel können dabei mit Röntgenmarkern, beispielsweise einer röntgenopaquen Beschichtung oder dergleichen, versehen sein, um die Überwachung des Positioniervorganges zu erleichtern.

Bei bevorzugten Weiterbildungen des erfindungsgemäßen Ballonkatheters ist das erste Führungsmittel zum Deformieren von Stützelementen eines im Bereich der Gefäßverzweigung angeordneten Stents ausgebildet. Hierdurch ist es möglich, mit dem ersten Führungsmittel die Stützelemente des Stents, die im Durchgangsbereich der Gefäßverzweigung angeordnet sind, ohne weitere Hilfsmittel so zu deformieren, daß der Durchgang zwischen den beiden Gefäßästen im wesentlichen frei ist.

Bei bevorzugten Varianten des erfindungsgemäßen Ballonkatheters sind die Kammern getrennt befüllbar ausgebildet, wodurch der Ballonkatheter in seinen Einsatzmöglichkeiten flexibler wird. So sind beispielsweise bestimmte Expansionsabläufe realisierbar, die mit einem herkömmlichen Einkammerballon nicht erzielt werden können.

Andere vorteilhafte Weiterbildungen der Erfindung sind in den Unteransprüchen gekennzeichnet bzw. werden nachstehend zusammen mit der Beschreibung der bevorzugten Ausführung der Erfindung anhand der Figuren näher dargestellt. Es zeigen:
- Figur 1: eine schematische Darstellung einer bevorzugten Ausführung des erfindungsgemäßen eingesetzten Ballonkatheters;
- Figur 2: eine schematische Darstellung eines erfindungsgemäßen Systems zum Aufweiten verengter Gefäße mit dem Ballonkatheter aus Figur 1;
- Figur 3: eine Abwicklung des Mantels einer Ausführung eines Stents für das System aus Figur 2;
- Figur 4: eine Abwicklung des Mantels einer weiteren Ausführung eines Stents für das System aus Figur 2.

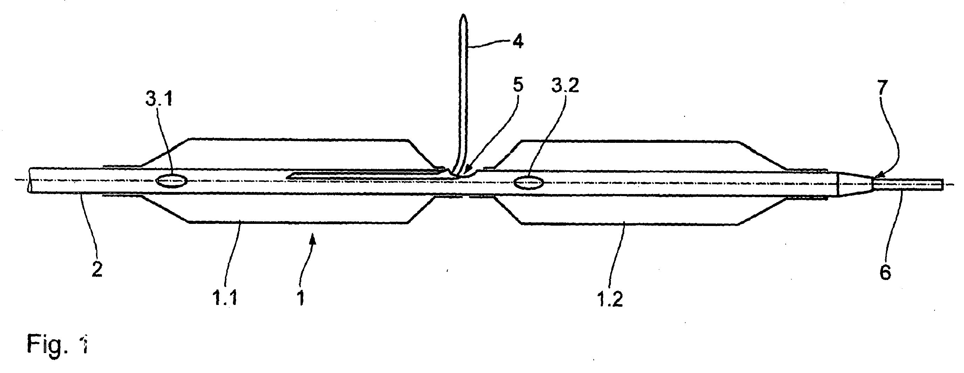

Figur 1 zeigt einen Ballonkatheter mit einem Ballon 1, der am distalen Ende eines Katheterschaftes 2 angeordnet ist. Der Ballon besteht dabei aus zwei in Längsrichtung des Ballonkatheters voneinander beabstandeten Kammern 1.1 und 1.2. Die Kammern 1.1 und 1.2 können getrennt über Befüllöffnungen 3.1 bzw. 3.2 zweier - nicht dargestellter - Zuleitungen im Katheterschaft 2 befüllt werden. Es versteht sich jedoch, daß die Befüllöffnungen bei anderen Varianten der Erfindung auch in einer gemeinsamen Zuleitungen ausgebildet sein können, um die beiden Kammern stets im wesentlichen gleichzeitig zu befüllen.

Im Katheterschaft 2 verläuft weiterhin ein - ebenfalls nicht dargestelltes - Führungslumen für ein als ersten Führungsdraht 4 ausgebildetes erstes Führungsmittel. Das Führungslumen weist dabei eine als Austrittsöffnung 5 ausgebildete seitliche Austrittseinrichtung auf, die am Katheterschaft 2 zwischen den beiden Kammern 1.1 und 1.2 des Ballons 1 angeordnet ist.

Der erste Führungsdraht 4 dient zum einen zur Unterstützung beim Positionieren des Ballons 1 im Bereich einer Gefäßverzweigung eines mittels des Ballons 1 aufzuweitenden Gefäßes. Hierzu kann der erste Führungsdraht 4 quer zur Längsrichtung des Ballonkatheters seitlich in den seitlich abzweigenden Gefäßast eingeführt werden. Um die Überwachung des Positioniervorganges zu erleichtern, ist der Führungsdraht an seinem distalen Ende mit einer röntgenopaquen Beschichtung versehen.

Durch den im Katheterschaft 2 geführten und erst zwischen den beiden Kammern 1.1 und 1.2 des Ballons 1 seitlich austretenden Führungsdraht ist sichergestellt, daß beim Expandieren des Ballons 1, anders als bei bekannten Varianten mit außen auf dem Ballon geführtem Führungsdraht, zwischen dem Ballon und der aufzuweitenden Gefäßwand bzw. einem zu expandierenden Stent nichts eingeklemmt wird. Hierdurch werden in diesen Bereichen bei den bekannten Ausführungen auftretende übermäßige lokale Belastungen bzw. Verformungen in dem aufzuweitenden Gefäß bzw. Stent verhindert, die zu Beschädigungen der aufzuweitenden Teile, gegebenenfalls sogar bis hin zum Reißen der Gefäßwand bzw. Bruch des Stents führen könnten.

Der Katheterschaft 2 weist noch ein weiteres - ebenfalls nicht dargestelltes - Führungslumen auf, in dem ein zweiter Führungsdraht 6 verläuft. Dieser zweite Führungsdraht 6 tritt am distalen Ende des Katheterschafts 2 durch eine distale Austrittsöffnung 7 aus diesem aus und dient zum Führen des Ballonkatheters in seiner Längsrichtung.

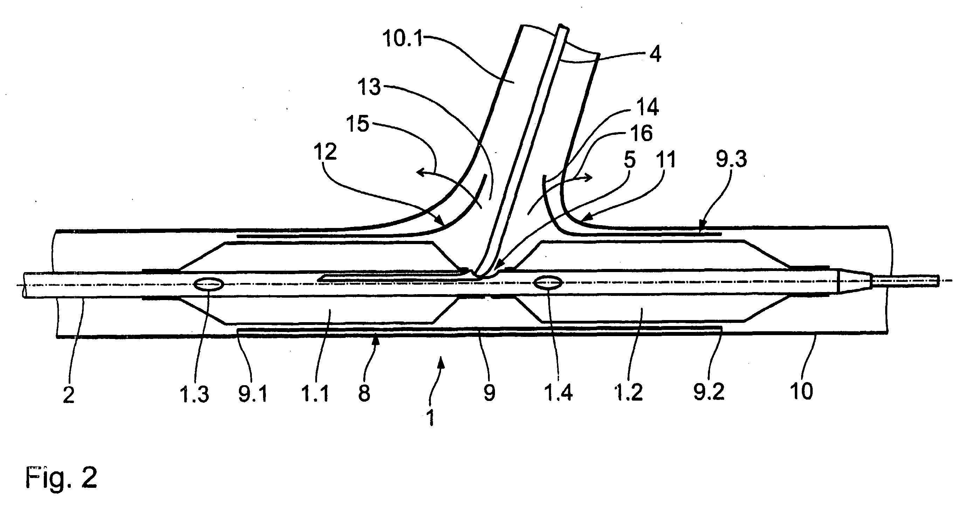

Figur 2 zeigt schematisch ein erfindungsgemäßes System zum Aufweiten verengter Gefäße im Bereich von Gefäßverzweigungen, das den Ballonkatheter aus Figur 1 und einen auf dem Ballon 1 angeordneten Stent 8 umfaßt.

Der Stent 8 weist einen rohrförmigen Mantel 9 mit einem proximalen Ende 9.1 und einem distalen Ende 9.2 auf. Er ist in einem in ein Blutgefäß 10 implantierten Zustand gezeigt. Er befindet sich dabei im Bereich einer Gefäßverzweigung 11, wobei sein proximales Ende 9.1 proximal der Gefäßverzweigung 11 und sein distales Ende 9.2 distal der Gefäßverzweigung 11 angeordnet ist.

Der Stent 8 weist einen Verzweigungsabschnitt 12 auf, der im gezeigten implantierten Zustand im Bereich der Gefäßverzweigung 11 angeordnet ist. Der Verzweigungsabschnitt 12 umfaßt zum Abstützen des Gefäßüberganges 11 ein langgestrecktes erstes Stützelement 13 und ein langgestrecktes zweites Stützelement 14. Im hier nicht dargestellten Ausgangszustand des Stents 8 liegen das erste Stützelement 13 und das zweite Stützelement 14 in der Mantelfläche 9.3 des Mantels 9. Das erste Stützelement 13 erstreckt sich dabei in einer ersten Richtung in den Verzweigungsabschnitt 12 hinein. Diese erste Richtung verläuft aus Richtung des proximalen Endes 9.1 des Stents 8 parallel zu dessen Längsachse. Das zweite Stützelement 14 erstreckt sich in einer zur ersten Richtung entgegengesetzten zweiten Richtung in den Verzweigungsabschnitt 12 hinein, d. h. aus Richtung des distalen Endes 9.2 parallel zur Längsachse des Stents 8.

Die Stützelemente 13 und 14 werden mittels des ersten Führungsdrahtes 4 des Ballonkatheters nach Art einer Saloon-Tür in Richtung der Pfeile 15 bzw. 16 radial aus der Mantelfläche 9.3 heraus in den abzweigenden Gefäßast 10.1 hinein gebogen. Im hier nicht dargestellten, fertig implantierten Zustand liegen das erste und zweite Stützelement 13 und 14 zur Abstützung des Gefäßüberganges 11 vollständig an dessen Wandung an.

Der Mantel 9 des Stents 8 kann dabei in beliebiger bekannter Weise ausgebildet sein. So kann er wie im gezeigten Beispiel mit einer plastisch deformierbaren Struktur, beispielsweise einer bekannten gitterartigen Stegstruktur, ausgebildet sein, die mittels des Ballons 1 des Ballonkatheters radial in den gezeigten aufgeweiteten Zustand verformt wird und nach Entfernen des Ballons 1 in diesem Zustand verbleibt, um das Gefäß 10 dauerhaft aufgeweitet zu halten.

Beim Einsatz des oben beschriebenen Systems zum Aufweiten verengter Gefäße wird vorzugsweise in der im folgenden beschriebenen Weise verfahren.

Zunächst wird der auf dem Ballon 1 sitzende Stent 8 im Bereich der Gefäßverzweigung 11 so positioniert, daß der Verzweigungsabschnitt 12 im Bereich der Gefäßverzweigung 11 angeordnet ist. Hierzu wird der Ballonkatheter mit einem Ballon 1 verwendet.

Der erste Führungsdraht 4 wird zur Unterstützung des Positioniervorganges durch eine entsprechende Öffnung im Bereich des Verzweigungsabschnittes 12 des hierbei noch im wesentlichen unverformten Stents 8 seitlich aus dem Stent heraus und in den abzweigenden Gefäßast 10.1 geführt.

Durch Befüllen der Kammern 1.1 und 1.2 über die Befüllöffnungen 1.3 bzw. 1.4 im Katheterschaft 2 mit einem druckbeaufschlagten Fluid werden diese expandiert und damit der auf dem Ballon 1 sitzende Stent 8 unter plastischer Verformung aufgeweitet, so daß er das Gefäß 10 nach Entfernen des Ballons 1 dauerhaft aufgeweitet hält.

Nach dem Expandieren des Mantels 9 des Stents 8 werden die Stützelemente 13 und 14 mittels des Führungsdrahtes 4 in Richtung der Pfeile 15 bzw. 16 radial aus der Mantelfläche 9.3 heraus unter plastischer Verformung in den abzweigenden Gefäßast 10.1 geklappt, um die Gefäßwandung im Bereich der Gefäßverzweigung 11 abzustützen.

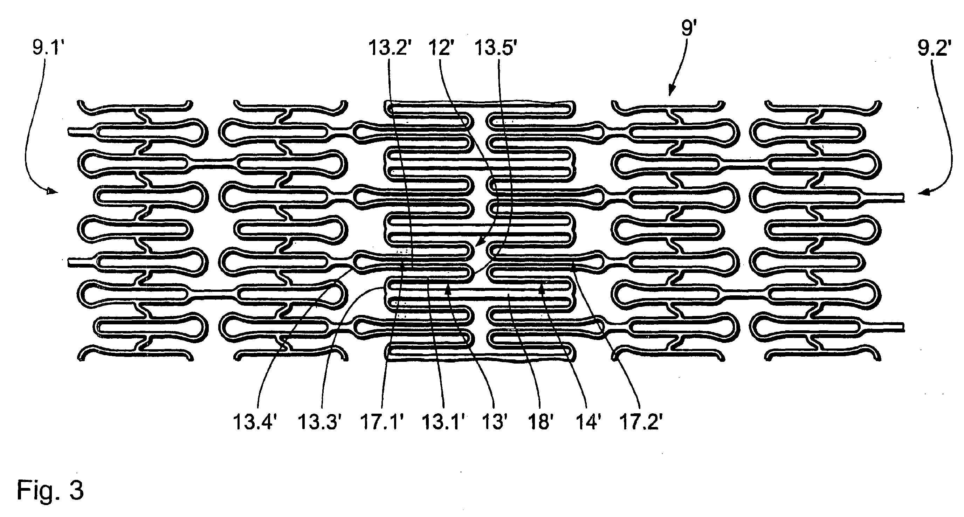

Figur 3 zeigt eine Abwicklung des Mantels 9' eines Stents 8' für das erfindungsgemäße System aus Figur 2, der aus einer Struktur stegartiger Elemente besteht. Der Mantel 9' weist dabei eine Anzahl in seiner Umfangsrichtung aneinander angrenzender Verzweigungsabschnitte 12' auf.

Der jeweilige Verzweigungsabschnitt 12' umfaßt ein erstes Stützelement 13', ein in der ersten Richtung, d. h. in Längsrichtung des Mantels 9', fluchtend dazu angeordnetes zweites Stützelement 14' sowie jeweils ein zu dem ersten bzw. zweiten Stützelement 13' bzw. 14' quer zur ersten Richtung, d. h. in Umfangsrichtung des Mantels 9', benachbart angeordnetes drittes Stützelement 17.1' bzw. 17.2'. Weiterhin wird der Verzweigungsabschnitt 12' in Umfangsrichtung des Mantels 9' zu beiden Seiten von einem in Richtung der Längsachse des Mantels 9' verlaufenden Steg 18' begrenzt.

Die Stützelemente 13', 14', 17.1' und 17.2' sind jeweils von sich im wesentlichen in Längsrichtung des Mantels 9' erstreckenden Stegelementen gebildet, die nach Art einer Haarnadel ausgebildet sind. Der Wendebereich bildet dabei jeweils das freie Ende des Stützelements 13', 14', 17.1' bzw. 17.2'.

Gestaltung und Anordnung der Stützelemente 13', 14', 17.1' bzw. 17.2' soll hier stellvertretend am Beispiel des Stützelements 13' erläutert werden. Die Schenkel 13.1' und 13.2' des Stützelements 13' sind so am Steg 18' bzw. am Mantel 9' angeschlossen, daß sich die Anschlußpunkte 13.3' und 13.4' bei der Expansion der Stentstruktur voneinander entfernen. Hierdurch wird zum einen das haarnadelartige Stützelement 13' in der Mantelfläche von der dem Wendebereich 13.5' abgewandten Seite her aufgebogen, woraus eine Vergrößerung des durch sie abstützbaren Gefäßbereichs resultiert. Zum anderen entfernen sich hierdurch auch die freien Enden der Stützelemente 13' und 17.1' bzw. 14' und 17.2' in Umfangsrichtung voneinander, woraus eine gleichmäßigere Verteilung der Stützelemente über den abzustützenden Gefäßübergang resultiert.

Der dem Steg 18' zugewandte erste Schenkel 13.1' ist dabei kürzer als der dem benachbarten Stützelement 17.1' zugewandte zweite Schenkel 13.2', so daß die Anschlußpunkte 13.3' und 13.4' in Längsrichtung des Mantels 9' voneinander beabstandet sind, Im implantierten Zustand ist somit bei um die Verbindungslinie der Anschlußpunkte in den abzweigenden Gefäßast geklappten Stützelementen 13', 14', 17.1' und 17.2' ein der ellipsenartigen Kontur des Gefäßübergangs im wesentlichen angepaßter Durchgang in das abzweigende Gefäß freigegeben. Hierdurch ist eine gleichmäßige Abstützung des Gefäßübergangs sichergestellt.

Aufgrund der Neigung der Verbindungslinie der Anschlußpunkte zur Umfangsrichtung des Mantels verlaufen die in den abzweigenden Gefäßast geklappten Stützelemente 13', 14', 17.1' bzw. 17.2' geneigt zur Längsrichtung des abzweigenden Gefäßastes, wodurch sich die Abstützung der Gefäßwandung durch die Stützelemente 13', 14', 17.1' bzw. 17.2' im Bereich des Gefäßüberganges in vorteilhafter Weise über einen größeren Umfangsbereich des abzweigenden Gefäßastes erstreckt. Dabei ist die Neigung zur Längsachse des abzweigenden Gefäßastes und damit der abgestützte Umfangsbereich umso größer, je stärker die Verbindungslinie der Anschlußpunkte zur Umfangsrichtung geneigt verläuft.

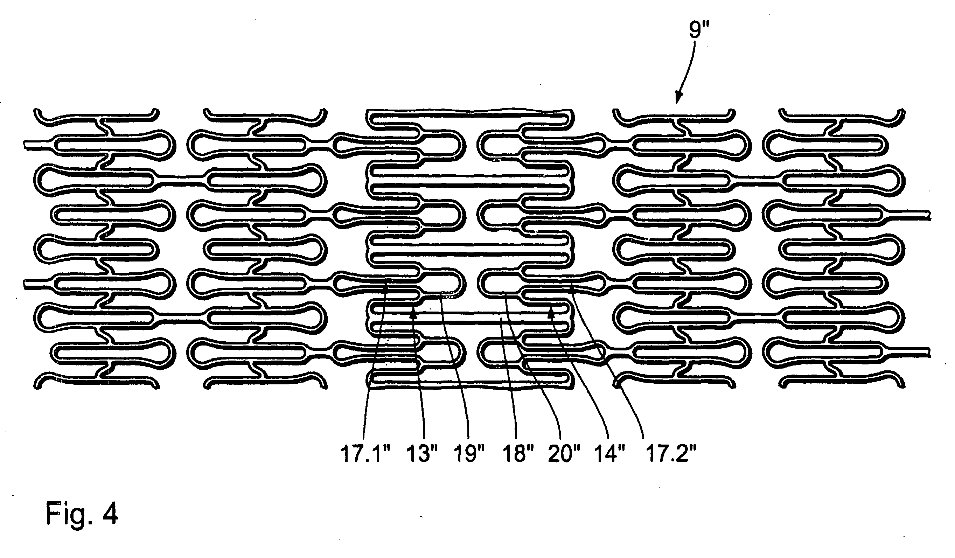

Figur 4 zeigt eine Abwicklung der Mantelfläche eines Stents 8'' für das erfindungsgemäße System aus Figur 2, die im wesentlichen der Ausführung aus Figur 3 gleicht, so daß hier lediglich auf Unterschiede eingegangen werden soll.

Der Unterschied liegt dabei darin, daß die freien Enden der Stützelemente 13'' und 17.1 " über ein stegartiges bogenförmiges Verbindungselement 19" und die freien Enden der Stützelemente 14'' und 17.2'' über ein stegartiges bogenförmiges Verbindungselement 20" verbunden sind. Das bogenförmige Verbindungselement 19'' bzw. 20'' weist dabei eine Bogenlänge auf, die ausreicht, um eine Abstandsänderung zwischen den freien Enden der Stützelemente 13" und 17.1 " bzw. 14" und 17.2'' bei der Expansion der Stentstruktur und dem anschließenden Umklappen der Stützelemente in den abzweigenden Gefäßast auszugleichen. Im umgeklappten Zustand stützt das Verbindungselement 19'' bzw. 20" dann in vorteilhafter Weise den Umfangsbereich der Gefäßwandung ab, der zwischen den freien Enden der Stützelemente 13" und 17.1'' bzw. 14" und 17.2" liegt.

## Patentansprüche

1. System zum Aufweiten verengter Gefäße, insbesondere verengter Koronargefäße, im Bereich von Gefäßverzweigungen, umfassend
einen Ballonkatheter mit einem Katheterschaft (2), an dessen distalem Ende wenigstens ein Ballon (1) und wenigstens ein in dessen Bereich angeordnetes erstes Führungsmittel (4) vorgesehen sind, wobei das erste Führungsmittel (4) zur Positionierung des Ballons (1) im Bereich einer Gefäßverzweigung (11) in den seitlich abzweigenden Gefäßast (10.1) quer zur Längsrichtung des Ballonkatheters einführbar ausgebildet ist und der Ballon (1) wenigstens zwei in Längsrichtung des Ballonkatheters voneinander beabstandete Kammern (1.1, 1.2) aufweist, zwischen denen das erste Führungsmittel (4) angeordnet ist, und
einen auf dessen Ballon (1) angeordneten Stent (8), der in dem an das erste Führungsmittel (4) angrenzenden Bereich zwischen den Kammern (1.1, 1.2) wenigstens einen am Umfang des Stentmantels (9; 9'; 9") angeordneten Verzweigungsabschnitt (12; 12'; 12") aufweist, der wenigstens ein zum Abstützen des Gefäßübergangs im Bereich einer Gefäßverzweigung (11) vorgesehenes, im wesentlichen radial aus der Mantelfläche herausklappbares erstes Stützelement (13; 13'; 13") umfaßt, und das erste Führungsmittel (4) zum Herausklappen des Stützelements (13; 13'; 13") aus der Mantelfläche ausgebildet ist.

2. System nach Anspruch 1, **dadurch gekennzeichnet, daß** das erste Führungsmittel des Ballonkatheters von einem ersten Führungsdraht (4) gebildet ist.

3. System nach Anspruch 2, **dadurch gekennzeichnet, daß** der erste Führungsdraht (4) in dem Katheterschaft (2) geführt ist, wobei der Katheterschaft (2) zwischen den beiden Kammern (1.1, 1.2) eine seitliche Austrittseinrichtung (5) für den ersten Führungsdraht (4) aufweist.

4. System nach einem der vorhergehenden Ansprüche, **dadurch gekennzeichnet, daß** ein zweiter Führungsdraht (6) zum Führen des Ballonkatheters in seiner Längsrichtung vorgesehen ist.

5. System nach Anspruch 4, **dadurch gekennzeichnet, daß** der zweite Führungsdraht (6) in dem Katheterschaft (2) geführt ist, wobei der Katheterschaft (2) an seinem distalen Ende eine distale Austrittseinrichtung (7) für den zweiten Führungsdraht (6) aufweist.

6. System nach einem der vorhergehenden Ansprüche, **dadurch gekennzeichnet, daß** die Kammern (1.1, 1.2) des Ballonkatheters getrennt befüllbar ausgebildet sind.

## Claims

1. A system for expanding constricted vessels, particularly constricted coronary vessels, in the area of vessel branches, comprising
a balloon catheter having a catheter shaft (2), on whose distal end at least one balloon (1) and at least one first guide means (4), which is positioned in its area, are provided, the first guide means (4), for positioning the balloon (1) in the area of a vessel branching (11), being implemented as insertable into the laterally branching vessel branch (10.1) transversely to the longitudinal direction of the balloon catheter, and the balloon (1) having at least two chambers (1.1, 1.2) spaced in the longitudinal direction of the balloon catheter, between which the first guide means (4) is positioned, and
a stent (8) positioned on this balloon (1), which has at least one branching section (12; 12'; 12") positioned around the circumference of the stent mantle (9; 9'; 9") in the area between the chambers (1.1, 1.2) adjoining the first guide means (4), which comprises a first support element (13; 13'; 13"), which may be folded essentially radially out of the mantle surface and is provided to support the vessel transition in the area of a vessel branching (11), and the first guide means (4) is implemented to fold the support element (13; 13'; 13") out of the mantle surface.

2. The system according to Claim 1, **characterized in that** the first guide means of the balloon catheter is formed by a first guide wire (4).

3. The system according to Claim 2, **characterized in that** the first guide wire (4) is guided in the catheter shaft (2), the catheter shaft (2) having a lateral exit device (5) for the first guide wire (4) between the two chambers (1.1, 1.2).

4. The system according to one of the preceding claims, **characterized in that** a second guide wire (6) is provided for guiding the balloon catheter in its longitudinal direction.

5. The system according to Claim 4, **characterized in that** the second guide wire (6) is guided in the catheter shaft (2), the catheter shaft (2) having a distal exit device (7) for the second guide wire (6) on its distal end.

6. The system according to one of the preceding claims, **characterized in that** the chambers (1.1, 1.2) of the balloon catheter are implemented so they may be filled separately.

## Revendications

1. Système d'élargissement de vaisseaux rétrécis, notamment de vaisseaux coronariens rétrécis, au niveau des ramifications de vaisseaux, comprenant
un cathéter à ballon comportant une tige de cathéter (2) à l'extrémité distale de laquelle il est prévu au moins un ballon (1) et au moins un premier moyen de guidage (4) disposé à ce niveau, le premier moyen de guidage (4) pour le positionnement du ballon (1) au niveau d'une ramification de vaisseau (11) étant réalisé de manière pouvoir être introduit dans la branche de vaisseau bifurquant latéralement (10.1) transversalement par rapport au sens longitudinal du cathéter à ballon et le ballon (1) présentant au moins deux chambres (1.1, 1.2) espacées l'une de l'autre dans le sens longitudinal du cathéter à ballon et entre lesquelles est disposé le premier moyen de guidage (4) et
un stent (8) disposé sur son ballon (1) et qui présente, dans la zone contiguë au premier moyen de guidage (4) entre les chambres (1.1, 1.2), au moins une section de ramification (12 ; 12' ; 12") disposée sur la circonférence de l'enveloppe du stent (9 ; 9' ; 9") et qui comprend au moins un premier élément support (13 ; 13' ; 13") prévu pour soutenir la transition de vaisseau au niveau d'une ramification de vaisseau (11) et rabattable sensiblement radialement depuis la surface d'enveloppe, et où le premier moyen de guidage (4) pour le rabattement de l'élément support (13 ; 13' ; 13") est réalisé dans la surface d'enveloppe.

2. Système selon la revendication 1, **caractérisé en ce que** le premier moyen de guidage du cathéter à ballon est constitué par un premier fil de guidage (4).

3. Système selon la revendication 2, **caractérisé en ce que** le premier fil de guidage (4) est guidé dans la tige de cathéter (2), la tige de cathéter (2) présentant, entre les deux chambres (1.1, 1.2), un dispositif latéral de sortie (5) pour le premier fil de guidage (4).

4. Système selon une des revendications précédentes, **caractérisé en ce qu'**il est prévu un deuxième fil de guidage (6) pour le guidage du cathéter à ballon dans son sens longitudinal.

5. Système selon la revendication 4, **caractérisé en ce que** le deuxième fil de guidage (6) est guidé dans la tige de cathéter (2), la tige de cathéter (2) présentant à son extrémité distale un dispositif distal de sortie (7) pour le deuxième fil de guidage (6).

6. Système selon une des revendications précédentes, **caractérisé en ce que** les chambres (1.1, 1.2) du cathéter à ballon sont réalisées de manière à être gonflables séparément.
